# EUROPEAN PATENT APPLICATION

(11) **EP 1 854 461 A1**
(43) Date of publication of application: **14.11.2007**
(21) Application number: 06714261.2
(22) Date of filing: 22.02.2006
(51) Int. Cl.: A61K 31/216, A61P 25/18, A23L 1/30

(54) **PROPHYLACTIC ANTISTRESS AGENT**

(30) Priority: 28.02.2005 JP 2005054532
(71) Applicant: Kao Corporation, Chuo-ku Tokyo 103-8210 (JP)
(72) Inventor: OCHIAI, Ryuji, kabane, Ichikai-machi, Haga-gun, Tochigi, 3213487 (JP); YAMASAKI, Yoshie, kabane, Ichikai-machi, Haga-gun, Tochigi, 3213497 (JP); FUJII, Akihiko, kabane, Ichikai-machi, Haga-gun, Tochigi, 3213497 (JP)
(74) Representative: HOFFMANN EITLE
(86) International application number: PCT/JP2006/303121
(87) International publication number: WO 2006/092992

(57) **Abstract**

The present invention relates to a prophylactic antistress agent and a central fatigue improver, which contain chlorogenic acids or pharmaceutically acceptable salts thereof as active ingredients. The present invention makes it possible to prevent and improve mental task fatigue, physical fatigue, central fatigue, central fatigue syndrome, overwork and the like.

## Description

### Field of the Invention

The present invention relates to an agent or food for the prevention of central fatigue or stress, which is a major cause of mental fatigue.

### Background of the Invention

Overwork death is defined as a sudden death attributable to excessively long hours of work, and this death is increasingly becoming a serious social problem. Severity of overwork death is well acknowledged medically and socially, but its developmental mechanism remains little elucidated (Non-Patent Documents 1 and 2).

Fatigue or stress has been cited as one of the causes leading to overwork death. Nevertheless, nothing has been known about a drug capable of ameliorating such a cause.

Chronic fatigue syndrome (CFS) is a disease characterized in that a healthy person is one day suddenly affected by a symptom of unknown cause (e.g., systemic malaise, low fever, headache, myalgia or psychoneurosis), and such a symptom continues over a long period of time, thereby making it almost impossible for the person to keep enjoying a healthy social life. This is a relatively new type of disease, which has been announced by CDC (Centers for Disease Control and Prevention) in 1988. In 1999, a research team of the Health, Labour and Welfare Ministry (ex. Ministry of Health and Welfare; team leader: Teruo Kitani) conducted an epidemiologic survey on the 4000 general people of a local community, aimed at investigating their fatigues (valid response number: 3,015). As a result, it was fond that 59.1% of the surveyed people complained of their fatigues, and that almost half of them suffered either from continued fatigue over more than six months, or from periodical fatigue. It was also found that nearly half of the people suffering from chronic fatigue were facing the deterioration of their working capacity, and 8 out of 3015 people (0.27%) were diagnosed to contract CFS under the CFS diagnostic criteria. Therefore, the treatment for the chronic fatigue due to an unknown cause is increasingly becoming a critical problem to be solved urgently, to medical institution having a role in primary care. Moreover, any chronic fatigue due to an unknown cause, including CFS, is becoming a serious social problem, from the viewpoint of economical loss, as well as from medical viewpoint.

With regard to chlorogenic acids, their antihypertensive action, autonomic nervous function-improving action, vascular endothelial function-improving action and the like have been reported, for example. However, there have been no reports regarding their antistress effect or their effect upon central fatigue (chronic fatigue) or overwork (e.g., Patent Documents 1 to 3).
[Patent Document 1] JP-A-2002-53464
[Patent Document 2] JP-A-2002-145765
[Patent Document 3] JP-A-2003-261444
[Non-Patent Document 1] Igaku no Ayumi (Progression of Medicine), Vol. 204, No. 5, pp. 362-364 (February 1st, 2003)
[Non-Patent Document 2] Nou 21 (Brain 21), Vol. 7, No. 1, pp. 41-45 (2004)

### Disclosure of the Invention

The present invention provides a prophylactic antistress agent, an agent for preventing and improving central fatigue, antistress food and drink, and food and drink for preventing and improving central fatigue, which contain chlorogenic acids or pharmaceutically acceptable salts thereof as active ingredients.
In addition, the present invention relates to use of chlorogenic acids or pharmaceutically acceptable salts thereof in production of a prophylactic antistress agent and a central fatigue improver.
Moreover, the present invention relates to a method of preventing and improving stress and central fatigue, containing administration of an effective amount of chlorogenic acids or pharmaceutically acceptable salts thereof.

### Brief Description of the Drawings

Figure 1 is a view showing action to extend a swimming time due to administration of chlorogenic acids (antistress level); and
Figure 2 is a view showing extension of a swimming time due to oral administration of chlorogenic acids (central fatigue improvement level).

### Detailed Description of the Invention

The present invention provides an agent and food, which have an antistress effect and a central fatigue-improving effect.

The present inventor evaluated various substances by carrying out a swimming test using water immersion load model rats, known as the model for evaluating fatigue level. As a result, it has been found that chlorogenic acids have an excellent prophylactic antistress effect and an excellent central fatigue-improving effect.

The present invention makes it possible to inhibit or prevent any of fatigue caused by stress, central fatigue, central fatigue syndrome and overwork.

Chlorogenic acids used in the present invention can be extracted from a natural product in which chlorogenic acids are contained, especially from a plant. Alternatively, said chlorogenic acids can be industrially produced by way of chemical synthesis.

The chlorogenic acids of the present invention include stereoisomers. In the present invention, pure stereoisomers or the mixtures thereof can be used. Specific examples of the chlorogenic acids used in the present invention include 3-caffeoylquinic acid, 4-caffeoylquinic acid, 5-caffeoylquinic acid, 3,4-dicaffeoylquinic acid, 3,5-dicaffeoylquinic acid, 4,5-dicaffeoylquinic acid, 3-feruloylquinic acid, 4-feruloylquinic acid, 5-feruloylquinic acid, and 3-feruloyl-4-caffeoylquinic acid (Nakabayashi et al., Coffee Baisen no Kagaku to Gijutsu (Chemistry and Techniques of Coffee Roasting), Kougaku Shuppan Co., Ltd., pp. 166-167).

By converting chlorogenic acids to salts thereof, their water solubility can be improved, and physiological effectiveness can be enhanced. The type of such salts is not limited, as long as they are pharmaceutically acceptable salts. Examples of basic substances used to form such salts include: alkaline metal hydroxides such as lithium hydroxide, sodium hydroxide, or potassium hydroxide; alkaline earth metal hydroxides such as magnesium hydroxide or calcium hydroxide; inorganic bases such as ammonium hydroxide; basic amino acids such as arginine, lysine, histidine, or ornithine; and organic bases such as monoethanolamine, diethanolamine, or triethanolamine. In particular, hydroxides of alkaline metals or alkaline earth metals are preferable. In the present invention, after such salts have been prepared, such salts may be added to a composition consisting of other components. Alternatively, chlorogenic acids and a salt-forming component may be added to the above composition separately, and salts may be then formed in the mixture.

Preferred examples of a natural product extract containing chlorogenic acids include extracts from plants such as coffee, cabbage, lettuce, artichoke, tomato, eggplant, potato, carrot, apple, pear, plum, peach, apricot, cherry, sunflower, Jew's mallow, sweet potato, Nandina domestica leaves, blueberry, or wheat.

The chlorogenic acids are preferably extracted from plant bodies such as raw coffee beans, Nandina domestica leaves, or unripe apple fruits. It is more preferably extracted from seeds of Coffee Arabica LINNE, using a warm ascorbic acid or citric acid aqueous solution, or hot water. As specific examples, "Flavor Holder" manufactured by T. Hasegawa Co., Ltd. is used as a raw coffee bean. Moreover, "Applephenon" manufactured by Nikka Whisky Distilling Co., Ltd. is used as an apple extract, and further, "Heliant" manufactured by Dainippon Ink and Chemicals, Inc. is used as a sunflower seed extract.

The term "prophylactic antistress agent" is used in the present invention to mean an agent for preventing fatigue caused by human stress, or an agent for preventing fatigue caused by the stress of brain. The term "fatigue" is used herein to mean a phenomenon whereby physical and mental performance is temporarily decreased when physical or mental load is continuously given. Such a decrease in performance is a qualitative or quantitative decrease in physical and mental work capacity. In addition, the term "fatigue" is used in the present invention to mean fatigue that is mainly caused by the stress of brain.

The term "central fatigue, central fatigue syndrome, and overwork" is used in the present invention to mean chronic fatigue and chronic fatigue syndrome (CFS).
Chronic fatigue is defined below. Depending on the cause of fatigue, cases where subjects answered to feel fatigue were divided into cases where such fatigue was caused by medical or psychological disease (fatigue due to disease), cases where such fatigue had a clear cause such as work or sports (clear cause), and cases where a cause of such fatigue was unknown (fatigue of unknown cause). Moreover, the period in which subjects felt fatigue was divided into 3 categories such as 5 months or less, 6 months or more, and a case where such fatigue had been felt from childhood. Among these cases, a case where fatigue had been felt for 6 months or more was defined as chronic fatigue. A case where a subject had not felt such fatigue for the past 1 year was defined as no fatigue. Idiopathic chronic fatigue and chronic fatigue syndrome-like fatigue are defined as follows. Among chronic fatigues due to unknown causes, fatigues wherein the fatigue level (performance status) is poor and it is 3 or greater, that is, wherein a subject cannot organize his/her social life or cannot work several days a month due to systemic malaise, and he/she needs rest at home, were selected. Moreover, when the subject's symptoms applied to 4 or more items out of the following symptom items: 1. a decrease in concentration power and thinking power; 2. sore throat; 3. swelling or pain of gular lymph node; 4. myalgia; 5. polyarticular pain; 6. headache; 7. vague sleep; and 8. systemic malaise continued for 24 hours after slight exertion, such symptoms were diagnosed to be chronic fatigue syndrome-like fatigue. Furthermore, when the subject's symptoms applied to 3 items out of the aforementioned symptom items and his/her fatigue level (performance status) was 3 or greater, such symptoms were diagnosed to be involved in idiopathic chronic fatigue.

Chlorogenic acids or the like used as active ingredients of the antistress agent and agent for preventing and improving central fatigue of the present invention may be directly administered. However, such chlorogenic acids may be preferably used in the form of pharmaceutically acceptable salts such as hydrochloride, together with drugs such as an excipient or carrier, and auxiliary components commonly used in the field of foods, such as lactose, sucrose, liquid sugar, honey, magnesium stearate, oxypropylcellulose, various types of vitamins, citric acid, malic acid, aromatic, or inorganic salts, so that it can be processed into a capsule, a tablet, a powder, a granule, a drinkable preparation, an injection, an infusion, etc.

In respect of a drinkable agent or food, other physiologically active components, minerals, vitamins, hormones, nutrients, flavoring agents and the like may be mixed therein, as needed. In addition, said agent and said food may be provided in the form of any of a green tea drink, an oolong tea drink, a black tea drink, a coffee drink and an isotonic drink.

The food of the present invention can be used with displays such as "this food has prophylactic antistress action," "to people who feel stress," "to people who feel physical fatigue," "to people who feel mental task fatigue," "to people who feel central fatigue," "to people who feel chronic fatigue," "to people who feel fatigue," etc.

When the prophylactic antistress agent or agent for preventing and improving central fatigue of the present invention is administered in the form of chlorogenic acids or pharmaceutically acceptable salts thereof, the dosage thereof is between 30 and 14,000 mg, more preferably between 50 and 10,000 mg, even more preferably between 200 and 7,600 mg, and far more preferably between 250 and 3,000 mg. In order to produce an antistress effect more effectively, it is preferable to continuously administer such an agent every day.

### Examples

### Example 1

SD rats (male, 7-week-old, n=4 to 12) were bred for 5 days in a cage wherein the water depth had been set at 1.5 cm (23±1°C) (1 rat/1 cage), so as to produce fatigue rats. It is to be noted that the water in such a cage was exchanged with fresh one every day. From the first day, a test sample or a normal saline solution used as a control was intraperitoneally administered to the rats once a day. At the same time, rats, which had been bred on a common bedding, were defined as normal rats, and from the first day, a normal saline solution was intraperitoneally administered to them once a day. After completion of the breeding for 5 days, a weight that was 8% of the body weight was attached to the tail portion of each rat on the 6^{th} day, and the rat was then allowed to swim in water of 23°C. The time required until the nose was submerged in water for 10 or more seconds was measured, and the thus measured time was evaluated as a fatigue level caused by stress.
For the test group, chlorogenic acid (CQA 300 mg/kg) was used. For the control group, a normal saline solution was used.

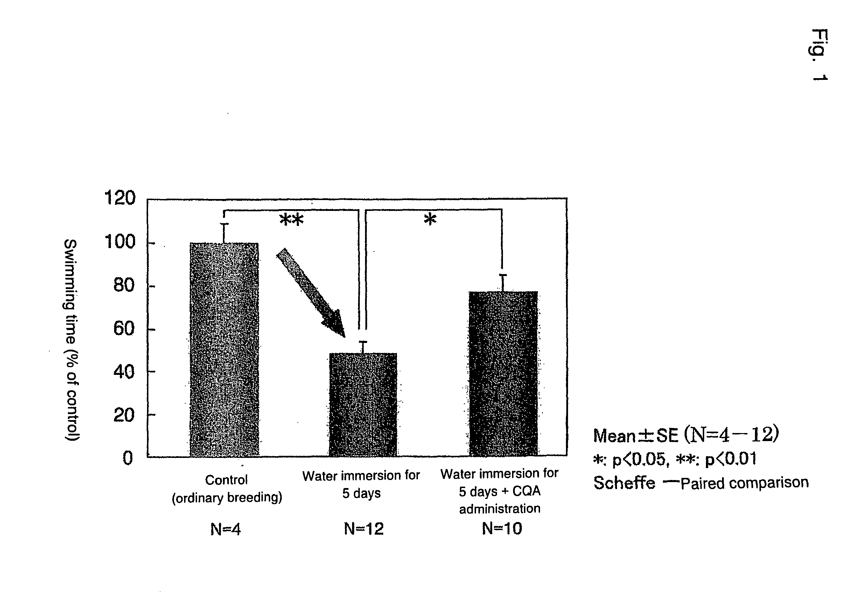

As a result, as shown in Figure 1, it was found that the fatigue level caused by stress is attenuated by pre-administration of chlorogenic acid.

### Example 2

A test sample or distilled water used as a control was administered in the form of drinking water to SD rats (male, 6-week-old, n=3 to 6). Thereafter, the rats were bred for 3 days in a cage wherein the water depth had been set at 1.5 cm (23±1°C) (1 rat/1 cage), so as to produce fatigue rats. It is to be noted that the water in such a cage was exchanged with fresh one every day. At the same time, rats, which had been bred on a common bedding, were defined as normal rats. After completion of the breeding for 3 days, a weight that was 8% of the body weight was attached to the tail portion of each rat on the 4^{th} day, and the rat was then allowed to swim in water of 23°C. The time required until the nose was submerged in water for 10 or more seconds was measured, and the thus measured time was evaluated as a fatigue level caused by central fatigue (chronic fatigue).
This model has been known as a central fatigue model (Non-Patent Document 1 and Neurosci. Lett. 352 (2003), 159-162).
For the test group, 0.25% (W/V) chlorogenic acid (370 mg/kg/day) was used. For the control group, distilled water was used.

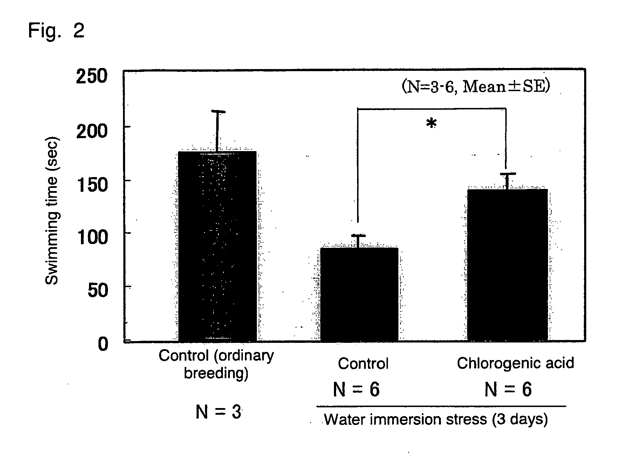

As a result, as shown in Figure 2, it was found that the fatigue level caused by central fatigue is attenuated by oral administration of chlorogenic acid.

## Claims

1. A prophylactic antistress agent comprising chlorogenic acids or pharmaceutically acceptable salts thereof as active ingredients.

2. The prophylactic antistress agent according to claim 1, wherein the prophylactic antistress agent is used to prevent a feeling of mental task fatigue or physical fatigue.

3. The prophylactic antistress agent according to claim 1 or 2, wherein the prophylactic antistress agent is a medicament or food.

4. An agent for preventing and improving central fatigue, central fatigue syndrome or overwork, which comprises chlorogenic acids or pharmaceutically acceptable salts thereof as active ingredients.

5. The agent according to claim 4, which is a medicament or food.

6. Use of chlorogenic acids or pharmaceutically acceptable salts thereof in production of a prophylactic antistress agent.

7. The use according to claim 6, wherein the prophylactic antistress agent is used to prevent a feeling of mental task fatigue or physical fatigue.

8. Use of chlorogenic acids or pharmaceutically acceptable salts thereof in production of an agent for preventing and improving central fatigue, central fatigue syndrome or overwork.

9. A method of preventing and improving stress, comprising administration of an effective amount of chlorogenic acids or pharmaceutically acceptable salts thereof.

10. The method according to claim 9, wherein the prevention and improvement of stress is to prevent and improve mental task fatigue or physical fatigue.

11. A method of preventing and improving central fatigue, central fatigue syndrome or overwork, comprising administration of an effective amount of chlorogenic acids or pharmaceutically acceptable salts thereof.
